# EUROPEAN PATENT SPECIFICATION

(11) **EP 1 518 551 B1**
(45) Date of publication and mention of the grant of the patent: **31.10.2007**
(21) Application number: 04255750.4
(22) Date of filing: 22.09.2004
(51) Int. Cl.: A61K 9/20, A61K 31/52

(54) **Solid dosage form comprising caffeine**
Solid Darreichungsform enthaltend Koffein
Forme d'administration solide comprenant cafeine

(30) Priority: 23.09.2003 US 671138
(43) Date of publication of application: 30.03.2005
(73) Proprietor: McNEIL-PPC, Inc., Skillman, New Jersey 08558 (US)
(72) Inventor: Shah, Indukumar G., North Wales PA 19454 (US); Szymczak, Christopher E., Marlton NJ 08053 (US); Osei, Anthony A., Harleysville PA 19438 (US); Pruss, Karl J., Bedminster PA 18910 (US)
(74) Representative: Mercer, Christopher Paul

(56) References cited:
- ES-A- 2 046 097
- GB-A- 1 287 431
- US-A- 4 049 803
- US-A- 4 420 483
- US-A- 4 943 565
- US-A- 5 409 709

## Description

The present invention relates to a solid pharmaceutical dosage form comprising caffeine and a cephalagic, such as an analgesic or an NSAID. The caffeine is in the form of uncoated particles having an average particle size of about 70 to 600 microns. The dosage form may be made by direct compression methods, and advantageously provides fast dissolution of the caffeine.

### Background of the Invention

The use of caffeine in combination with other active ingredients in pharmaceutical dosage forms is known. For example, Canadian Patent No. 1,336,687 discloses a process for preparing tablets containing ibuprofen, acetaminophen (APAP), and caffeine. The tablets are made by first preparing wet granulations of each ingredient, combining the three granulations into a mixture, and tabletting the mixture. This patent teaches that separate wet granulation of the ingredients is necessary and preferred over direct compression of the three active ingredients together.

U.S. Patent No. 4,943,565 relates to an analgesic tablet containing aspirin, APAP, caffeine and hydroxypropyl cellulose. The caffeine was cogranulated with the other active ingredients using hydroxypropyl cellulose.

U.S. Patent No. 4,486,436 relates to compositions comprising caffeine together with analgesics and/or non-steroidal anti-inflammatory drugs (NSAID's). The '436 patent discloses that caffeine in anhydrous powder form, or any salt or derivative of caffeine or any compounded mixture thereof which is non-toxic, pharmaceutically acceptable, and capable of hastening and enhancing an analgesic or anti-inflammatory response when employed may be used.

Applicants have now discovered that when caffeine of a certain type is employed in dosage forms, the caffeine unexpectedly dissolves from the dosage form faster. In particular, dosage forms comprising caffeine in the form of uncoated particles having an average particle size of about 70 to 600 microns provide fast caffeine dissolution rates. Typically, at least 95 % of the caffeine dissolves in less than 5 minutes, when measured by the United States Pharmacopoeia (USP), Type II Apparatus (Paddles) set at 50 rpm.

### Summary of the Invention

The invention provides a solid pharmaceutical dosage form comprising caffeine and a cephalagic, wherein said caffeine is in the form of uncoated particles having an average particle size of about 70 to 600 microns.

The invention also provides a process for making a solid, pharmaceutical dosage form, which comprises dry blending caffeine and a cephalagic into a blend, wherein said caffeine is in the form of uncoated particles having an average particle size of about 70 to 600 microns, and compressing the blend.

### Detailed Description of the Invention

The dosage form comprises caffeine and one or more cephalagics. The cephalagic may be selected for example from analgesics, NSAID's, decongestants, antihistamines, and other agents known to treat headache and headache-related disorders. In one embodiment, the cephalagic is an analgesic selected from acetaminophen (APAP) and tramadol. In another embodiment, the cephalagic is an NSAID selected from ibuprofen and ketoprofen. In a further embodiment, the cephalagic is an antihistamine selected from diphenhydramine, chlorpheniramine and doxcylamine. Preferably, the cephalagic is selected from the group consisting of acetaminophen, ibuprofen, ketoprofen, chlorpheniramine, diphenhydramine and doxylamine.

In one embodiment, the cephalagic is in the form of a granulation. The average particle size of such granulation is preferably in the range of about 100 to about 400 microns. This particle size range allows for adequate flow during processing and content uniformity.

The amount of caffeine used is typically in the range of about 5 mg to about 400 mg, preferably about 15 mg to about 200 mg per unit dose. The amount of cephalagic such as acetaminophen is typically in the range of about 1 mg to about 750 mg, preferably about 2 mg to about 500 mg per unit dose.

The caffeine is in the form of uncoated, ungranulated, larger particles ("granular" in morphology) having an average particle size of about 70 to 600, preferably 180 to 500, more preferably 200 to 500, microns. This is critical to the invention. Applicants have discovered that use of uncoated, ungranulated caffeine of this relatively larger particle size results in faster dissolution rates than that of powdered caffeine, even when disintegrants are used with powdered caffeine as part of a wet granulation process. The surfaces of the caffeine particles according to the invention are substantially free, preferably free, of polymeric binders and coating materials. One commercially available form of such caffeine may be obtained from BASF under the designation anhydrous caffeine granular, 0.2/0.5.

The dosage form is solid. In one embodiment, the dosage form is a compressed tablet or caplet. The dosage form may also be uncoated or coated with conventional coating materials. The dosage form may comprise conventional additives and excipients useful with solid dosage forms, such as fillers, including water soluble compressible carbohydrates such as sucrose, mannitol, sorbitol, maltitol, xylitol, erythritol, lactose, and mixtures thereof; conventional dry binders including cellulose, cellulosic derivatives, polyvinyl pyrrolidone, starch, modified starch, and mixtures thereof; sweeteners including aspartame, acesulfame potassium, sucralose, saccharin and mixtures thereof; disintegrants such as microcrystalline cellulose, starch, sodium starch glycolate, crosslinked polyvinylpyrrolidone, crosslinked carboxymethylcellulose; and lubricants, such as magnesium stearate, stearic acid, talc, vegetable oils and waxes. The dosage form may also incorporate pharmaceutically acceptable adjuvants, including for example preservatives, flavors, acidulants, antioxidants, glidants, surfactants, and coloring agents.

In one embodiment of the invention, the dosage form comprises a directly compressed blend of caffeine in the form of uncoated particles having an average particle size of about 200 to 500 microns and APAP in the form of a granulation having an average particle size of 100 to 400 microns, along with a lubricant such as magnesium stearate or stearic acid.

Advantageously, the dosage form may be made by dry, direct compression methods. In particular, the dosage form may be made by dry blending caffeine in the form of uncoated particles having an average particle size of about 70 to 600 microns and a cephalagic into a blend, and compressing the blend.

Typically, at least 95 % of the caffeine dissolves from a dosage form of the invention in less than 5 minutes, when measured by USP Type II Apparatus (Paddles) at 50 rpm. This is unexpected in that the caffeine used is of a relatively large particle size as compared to powdered caffeine, which has a typical average particle size of about 50 microns. At the same time, uncoated caffeine according to the invention has been found to meet the content uniformity required by USP standards with commonly used cephalagics having particle sizes of from about 100 to about 400 microns. This is useful to achieve faster therapeutic action of the caffeine, which is desirable when treating symptoms such as headache, migraines, or in cases where side effects such as sedation are undesirable.

The following non-limiting example further illustrates the invention.

### Example

A dosage form according to the invention was made by blending the following ingredients in dry form. The blend was directly compressed into caplets using a Fette 3090 tablet press set at 200, 360 or 400 thousand tablets per hour (61 station). The compressed caplets (with an average hardness of 13 KN or KiloNewtons of force) were coated with a mixture of hydroxypropylmethyl cellulose (HPMC E-5) and castor oil to a 2% weight gain. The caffeine was in the form of uncoated particles having an average particle size of 300-400 microns, commercially available from BASF as anhydrous caffeine, 0.2/0.5.

| Ingredient | mg/caplet |
|---|---|
| APAP granulation | 600.00* |
| Caffeine | 65.00 |
| Microcrystalline cellulose PH-101 | 66.86 |
| Sodium starch glycolate | 3.70 |
| Magnesium stearate | 4.44 |
| Total | 740.00 |

| | |
|---|---|
| *APAP granulation (per 600 mg): 40 mg starch, 500 mg APAP, 40 mg powdered cellulose, 10 mg sodium starch glycolate, and 10 mg pregelatinized starch. | |

The caplets were tested for caffeine and APAP dissolution rates using USP, Type II Apparatus (Paddles) set at 50 rpm and 100 rpm. Commercially available EXCEDRIN product, made by wet granulating caffeine and APAP together and compressing into dosage forms, was also tested for dissolution. In addition, commercially available NODOSE product, containing 200 mg of caffeine as the sole active ingredient, was tested for dissolution at a 50 rpm paddle speed.

The caplets according to the invention demonstrated a significantly faster rate of caffeine dissolution at earlier time points than both the EXCEDRIN and NODOSE products. The average percent of caffeine released from the two formulations is given in the Table below.

| **50 RPM/Paddle** | | | | | **100 RPM/Paddle** | | | | 50 RPM/Paddle |
|---|---|---|---|---|---|---|---|---|---|
| Time (min) | Coated Caplet according to the invention | | EXCEDRIN | | Coated Caplet according to the invention | | EXCEDRIN | | NODOSE |
| | % Caffeine | % APAP | % Caffeine | % APAP | % Caffeine | % APAP | % Caffeine | % APAP | % Caffeine |
| 5 | 86 | 88 | 36 | 41 | 102 | 95 | 50 | 51 | 19 |
| 10 | 101 | 101 | 72 | 78 | 104 | 99 | 90 | 89 | 34 |
| 15 | 102 | 101 | 91 | 95 | 104 | 99 | 101 | 99 | 52 |
| 30 | 101 | 101 | 96 | 100 | 104 | 99 | 101 | 100 | 94 |
| 60 | 101 | 101 | 95 | 100 | 104 | 100 | 101 | 100 | 104 |

## Claims

1. A solid pharmaceutical dosage form comprising caffeine and a cephalagic, wherein said caffeine is in the form of uncoated particles having an average particle size of about 70 to 600 microns.

2. The dosage form of claim 1, wherein the cephalagic is an analgesic, non-steroidal anti-inflammatory drug, decongestant or antihistamine.

3. The dosage form of claim 2, wherein the cephalagic is acetaminophen, ibuprofen, ketoprofen, chlorpheniramine, diphenhydramine or doxcylamine.

4. The dosage form of any one of claims 1 to 3, wherein the cephalagic is in the form of a granulation.

5. The dosage form of claim 4, wherein the granulation has an average particle size of 100 to 400 microns.

6. The dosage form of any one of claims 1 to 5 in the form of a directly compressed tablet.

7. The dosage form of any one of claims 1 to 6, wherein at least 95 % of the caffeine dissolves within 5 minutes, when measured by USP, Type II Apparatus (Paddles) set at 50 rpm.

8. A process for making a solid, pharmaceutical dosage form, which comprises dry blending caffeine and a cephalagic into a blend, wherein said caffeine is in the form of uncoated particles having an average particle size of 70 to 600 microns, and compressing the blend.

9. The process of claim 8, which is used to produce a doage form as defined in any one of claims 1 to 7.

## Patentansprüche

1. Feste pharmazeutische Dosierungsform, die Coffein und ein Kopfschmerzmittel umfaßt, wobei das Coffein in Form unbeschichteter Teilchen mit einer durchschnittlichen Teilchengröße von etwa 70 bis 600 Mikrons vorliegt.

2. Dosierungsform nach Anspruch 1, **dadurch gekennzeichnet, daß** das Kopfschmerzmittel ein Analgetikum, nicht-steroider entzündungshemmender Arzneistoff, Abschwellungsmittel oder Antihistaminikum ist.

3. Dosierungsform nach Anspruch 2, **dadurch gekennzeichnet daß** das Kopfschmerzmittel Acetaminophen, Ibuprofen, Ketoprofen, Chlorpheniramin, Diphenhydramin oder Doxcylamin ist.

4. Dosierungsform nach einem der Ansprüche 1 bis 3, **dadurch gekennzeichnet, daß** das Kopfschmerzmittel in Form eines Granulats vorliegt.

5. Dosierungsform nach Anspruch 4, **dadurch gekennzeichnet, daß** das Granulat eine durchschnittliche Teilchengröße von 100 bis 400 Mikrons aufweist.

6. Dosierungsform nach einem der Ansprüche 1 bis 5 in Form einer direkt verpreßten Tablette.

7. Dosierungsform nach einem der Ansprüche 1 bis 6, **dadurch gekennzeichnet, daß** wenigstens 95% des Coffeins sich innerhalb von 5 Minuten lösen, wenn gemessen mit einer USP-Typ-II-Apparatur (Schaufeln), eingestellt auf 50 UPM.

8. Verfahren zur Herstellung einer festen, pharmazeutischen Dosierungsform, welches das Trockenvermischen von Coffein und einem Kopfschmerzmittel zu einem Gemisch, wobei das Coffein in Form unbeschichteter Teilchen mit einer durchschnittlichen Teilchengröße von 70 bis 600 Mikrons vorliegt, und Verpressen des Gemisches umfaßt.

9. Verfahren nach Anspruch 8, das verwendet wird, um eine Dosierungsform herzustellen, wie definiert in einem der Ansprüche 1 bis 7.

## Revendications

1. Forme pharmaceutique solide comprenant de la caféine et un anticéphalalgique, dans laquelle ladite caféine se présente sous forme de particules non enrobées ayant une taille de particule moyenne d'environ 70 à 600 microns.

2. Forme selon la revendication 1, dans laquelle l'anticéphalalgique est un analgésique, médicament anti-inflammatoire non stéroïdien, décongestionnant ou anti-histaminique.

3. Forme selon la revendication 2, dans laquelle l'anticéphalalgique est l'acétaminophène, l'ibuprofène, le kétoprofène, la chlorphéniramine, la diphénhydramine ou la doxcylamine.

4. Forme selon l'une quelconque des revendications 1 à 3, dans laquelle l'anticéphalalgique se présente sous la forme d'un granulé.

5. Forme selon la revendication 4, dans laquelle le granulé a une taille de particule moyenne de 100 à 400 microns.

6. Forme selon l'une quelconque des revendications 1 à 5, sous la forme d'un comprimé obtenu par compression directe.

7. Forme selon l'une quelconque des revendications 1 à 6, dans laquelle la caféine se dissout à hauteur d'au moins 95% en l'espace de 5 minutes, mesuré au moyen d'un appareil USP de type II (palettes) réglé à 50 tr/min.

8. Procédé de production d'une forme pharmaceutique solide, qui comprend le mélangeage à sec de caféine et d'un anticéphalalgique pour donner un mélange dans lequel ladite caféine se présente sous la forme de particules non enrobées ayant une taille de particule moyenne de 70 à 600 microns, et la compression du mélange.

9. Procédé selon la revendication 8, qui est utilisé pour produire une forme telle que définie dans l'une quelconque des revendications 1 à 7.
